# EUROPEAN PATENT SPECIFICATION

(11) **EP 1 749 018 B1**
(45) Date of publication and mention of the grant of the patent: **22.10.2014**
(21) Application number: 05739183.1
(22) Date of filing: 25.04.2005
(51) Int. Cl.: C07K 1/00, A61K 38/00, A61K 47/36

(54) **METHODS AND KITS FOR STABILISING, PROTECTING AND SOLUBILISING PROTEINS**
VERFAHREN UND KITS ZUR STABILISIERUNG, ZUM SCHUTZ UND ZUR SOLUBILISIERUNG VON PROTEINEN
PROCEDES ET KITS POUR STABILISER, PROTEGER ET SOLUBILISER DES PROTEINES

(30) Priority: 23.04.2004 GB 0409088; 13.09.2004 WO PCT/GB2004/050009; 29.09.2004 GB 0421613; 15.03.2005 GB 0505229
(43) Date of publication of application: 07.02.2007
(73) Proprietor: Expedeon Limited, Cambridgeshire CB2 4AT (GB)
(72) Inventor: JONES, Daniel Brian, Cambridge Cambridgeshire CB2 4AT (GB); LANCKRIET, Heikki, Cambridge Cambridgeshire CB2 4AT (GB)
(74) Representative: Schiweck, Weinzierl & Koch
(86) International application number: PCT/GB2005/050057
(87) International publication number: WO 2005/103067

(56) References cited:
- EP-A- 0 267 015
- EP-A- 0 410 207
- WO-A-97/41885
- WO-A-03/040398
- WO-A-2005/026196
- GB-A- 2 405 872
- US-A- 4 824 938
- HINRICHS W L J ET AL: "Inulin glasses for the stabilization of therapeutic proteins" INTERNATIONAL JOURNAL OF PHARMACEUTICS, AMSTERDAM, NL, vol. 215, no. 1-2, 14 March 2001 (2001-03-14), pages 163-174, XP002318384 ISSN: 0378-5173
- SIVAKAMA SUNDARI C ET AL: "Artificial chaperoning of insulin, human carbonic anhydrase and hen egg lysozyme using linear dextrin chains - a sweet route to the native state of globular proteins" FEBS LETTERS, ELSEVIER SCIENCE PUBLISHERS, AMSTERDAM, NL, vol. 443, no. 2, 29 January 1999 (1999-01-29), pages 215-219, XP004259122 ISSN: 0014-5793
- MACHIDA^A S ET AL: "Cycloamylose as an efficient artificial chaperone for protein refolding" FEBS LETTERS, ELSEVIER SCIENCE PUBLISHERS, AMSTERDAM, NL, vol. 486, no. 2, 8 December 2000 (2000-12-08), pages 131-135, XP004337827 ISSN: 0014-5793
- ARAKAWA T ET AL: "Factors affecting short-term and long-term stabilities of proteins" ADVANCED DRUG DELIVERY REVIEWS, AMSTERDAM, NL, vol. 46, no. 1-3, 1 March 2001 (2001-03-01), pages 307-326, XP002318382 ISSN: 0169-409X
- STEVENS ET AL: "Polymeric Surfactants based on Inulin", BIOMACROMOLECULES, AMERICAN CHEMICAL SOCIETY; US, vol. 2, no. 4, 10 June 2001 (2001-06-10), pages 1256-1259, XP009131610, ISSN: 1525-7797, DOI: 10.1021/BM015570L

## Description

### Technical Field

The present invention relates to methods, kits and reagents for stabilising protein conformation, protecting proteins from aggregation and/or degradation, and/or for improving protein solubility.

### Background to the Invention

The biological function of a protein is dependent on its three dimensional structure. Proteins are formed as linear chains of amino acids, termed polypeptides. *In vivo,* in the appropriate conditions within the cell, the linear polypeptide chain is folded, a process which may be assisted by proteins called chaperones. A mature folded protein has an active three dimensional conformation, known as the native structure. The structure depends on weak forces such as hydrogen bonding, electrostatic and hydrophobic interactions. These forces are affected by the protein environment, so changes in the environment may cause structural disruption resulting in denaturation and/or degradation of the protein and loss of function. Such problems are encountered during processing of proteins and on storage of proteins.

The production of proteins by genetic engineering often results in the accumulation of non-active protein aggregates as inclusion bodies. After isolation and purification from the host cells, proteins or inclusion bodies have to be unfolded (denatured) and subsequently refolded (renatured) so that the proteins regain their native structure and bioactivity.

Traditional protein folding methods involve denaturant dilution or column-based approaches. Denatured protein is commonly refolded by diluting the denaturant. This induces a hydrophobic collapse of the protein molecule and in doing so the protein shields its hydrophobic patches in the core of the molecule. Unfortunately, on hydrophobic collapse, proteins do not always form the native bioactive conformation; two competing reactions occur: refolding and aggregation. It is suggested that a driver for protein aggregation is hydrophobic amino acid residues exposed at the surface. Aggregation is undesirable and reduces the yield of functional, native protein. Refolding is known as a first order reaction (Rate = K * [prot]), but the aggregation reaction is favoured over refolding at high concentration as it is a higher order reaction (n) (Rate = K' * [prot]ⁿ). The proportion of protein refolding:protein aggregation is strongly dependant on the protein concentration. At process scale this concentration dependency can result in increased aggregation, and thus reduced refolding yield. This can be due to imperfect mixing patterns in the protein solution. Processing large protein molecules is particularly difficult as they diffuse more slowly than the smaller denaturant molecules, thus creating micro-environments containing high localised protein concentrations and low denaturant concentration, that is, an environment that favours protein aggregation over protein refolding.

For proteins with disulphide bonds the native protein often needs to be "matured"; during maturation, non-aggregated monomeric protein molecules with non-native disulphide bonds may be created initially; then, using a protein specific redox couple, the disulphide bonds shuffle and the protein matures to a functional protein molecule with native disulphide bonds.

Refolding processes usually involve dispersing the denatured protein molecules in a buffer in the presence of "refolding aids" to enhance renaturation. Folding aids usually increase the solubility of the folding intermediates and/or change the relative reaction rates of the folding and aggregation reactions. Polyethylene glycol and various sugars, e.g. sucrose, glucose, N-acetylglucosamine, and detergents, e.g. Chaps, Tween, SDS, Dodecylmaltoside have been employed as refolding aids (De Bernadez Clark (1998) Current Opinion Biotechnol. 9, 157-163).

α, β and γ cyclodextrins (CDs) have been reported to be useful in stabilisation, solubilisation and affinity purification of certain enzymes, but both the nature of the interactions between these CDs and proteins, and their effect on bioactivity remain unclear. α, β and γ-cyclodextrins have been used as artificial chaperones to aid protein refolding in both detergent-free (Sharma et al, EP 0 871 651, US 5,728,804) and detergent-containing refolding environments (Gellman & Rozema, US 5,563,057). Cyclodextrins are cyclic oligosaccharides composed of multiple glucose residues. They are classified according to the number of sugar residues within the ring structure, α-cyclodextrin has 6 glucose residues, β-cyclodextrin has 7 glucose residues and γ-cyclodextrin has 8 glucose residues. Cyclodextrins can be modified by derivatisation to produce derivatives.

The inner cavity of cyclodextrins is hydrophobic whereas the outer surface is hydrophilic. The hydrophobic interior is capable of encapsulating poorly soluble drugs. The hydrophilic exterior assists in solubilisation, so cyclodextrins are useful adjuncts in pharmaceutical formulation.

EP 0 094 157 & US 4,659,696 (Hirai et al) describe the use of α-, β-, and γ-cyclodextrin derivatives in pharmaceutical compositions consisting essentially of a physical mixture of a hydrophilic, physiologically active (folded, native) peptide and a cyclodextrin derivative, the composition being a uniform mixture in dosage form.

EP 0 437 678 B1, US 5,730,969 and US 5,997,856 (Hora) describe methods for the solubilisation and/or stabilisation of polypeptides, especially proteins, using specified cyclodextrin derivatives: hydroxypropyl, hydroxyethyl, glucosyl, maltosyl, and maltotriosyl derivatives of β- and γ-cyclodextrin; the hydroxypropyl-β-cyclodextrin derivative being preferred. Also disclosed are aqueous and lyophilised compositions comprising a polypeptide, optionally a protein, and the above specified cyclodextrin derivatives.

EP 0 871 651 & US 5,728,804 (Sharma et al) are concerned with a method for renaturing an unfolded or aggregated protein in a detergent-free aqueous medium with an amount of a cyclodextrin effective to renature said unfolded or aggregated protein. In this instance, the protein is present at a low concentration selected to minimise aggregation, preferably at around 0.05 mg/ml, and spontaneously refolds in a refolding buffer containing cyclodextrin. After refolding the cyclodextrin is removed by dialysis.

US 5,563,057 (Gellman & Rozema) describes a method for refolding an enzyme from a misfolded configuration to a second native active configuration by adding a detergent having a linear alkyl non-polar portion, e.g. CTAB and Triton^{®}-X 100 (Octoxynol-9), to misfolded enzyme to form an enzyme-detergent complex, which is then contacted with a cyclodextrin to allow the enzyme to assume a second active conformation.

Hinrichs et al (2001). International Journal of Pharmaceutics, 215, 163-174, describes the use of non-derivatised inulins, inulin SC 95 (DPn/DPw = 5.5/6.0), inulin RS (DPn/DPw = 14.2/19.4), and inulin EXL 608 (DPn/DPw 23.0/26.2) to protect alkaline phosphatase from degradation during freeze drying and subsequent storage of the dried protein.

WO 96/41870 (Gombac et al) describe frozen, dried or lyophilised hydrosoluble collagenase compositions containing isomalt and/or inulin (non-derivatised) to stabilise the collagenase.

EP 0267015 describes a method of stabilising a medicinal composition containing a polypeptide growth factor having mitogenic activity against loss of biological activity in the presence of moisture by the use of a stabilizing amount of a water-soluble polysaccharide, preferable a cellulose derivative, such as methylcellulose or a hydroxyl alkylcellulose, such as hydroxypropyl methylcellulose.

Inulins are D-fructans, generally consisting of chains of polyfructose in which the fructose units are connected to each other mostly or exclusively by beta (2-1) linkages. Inulin occurs in nature, in general, as a polydisperse mixture of polyfructose chains most of which are ending in one glucosyl unit. Inulin can be obtained from bacterial synthesis, extracted from plants or can be made *in vitro* by enzymatic synthesis starting from sucrose. Inulin produced by bacteria is more branched than inulin from plant origin and commonly has a higher molecular weight (ranging from about 2,000 up to about 20,000,000), whereas inulin from plant origin is generally composed of linear or slightly branched polyfructose chains, or mixtures thereof, with a molecular weight commonly ranging from about 600 to about 20,000.

Inulin can be represented, depending from the terminal carbohydrate unit, by the general formulae GF.n or F.n, wherein G represents a glucosyl unit, F a fructosyl unit, and n is an integer representing the number of fructosyl units linked to each other in the carbohydrate chain. The number of saccharide units (fructose and glucose units) in one inulin molecule, i.e. the value of n in the formulae above, is referred to as the degree of polymerisation, represented by (DP). Often, the parameter (number) average degree of polymerisation, represented by (DP), is used too, which is the value corresponding to the total number of saccharide units (G and F units) in a given inulin composition divided by the total number of inulin molecules present in said inulin composition, without taking into account the possibly present monosaccharides glucose (G) and fructose (F), and the disaccharide sucrose (GF). The average degree of polymerisation (DP) can be determined, for example, by the method described by L. De Leenheer (Starch, 46 (5), 193-196, (1994), and Carbohydrates as Organic Raw Materials, Vol. III, p. 67-92, (1996)).

Inulin is commonly prepared from plant sources, mainly from roots of Chicory *(Cichorium intybus*) and from tubers of Jerusalem artichoke (*Helianthus tuberosus*), in which inulin can be present in concentrations of about 10 to 20% w/w on fresh plant material. Inulin from plant origin is usually a polydisperse mixture of linear and slightly branched polysaccharide chains with a degree of polymerisation (DP) ranging from 2 to about 100. In accordance with known techniques, inulin can be readily extracted from said plant parts, purified and optionally fractionated to remove impurities, mono- and disaccharides and undesired oligosaccharides, in order to provide various grades of inulin, e.g. as described in EP 0 769 026 and EP 0 670 850.

Inulin is commercially available, typically with a (DP) ranging from about 6 to about 40. Inulin from chicory is for example available as RAFTILINE^{®} from Orafti (Tienen, Belgium) in various grades. Typical RAFTILINE^{®} grades include RAFTILINE^{®} ST (with a (DP) of about 10 and containing in total up to about 8% by weight glucose, fructose and sucrose), RAFTILINE^{®} LS (with a (DP) of about 10 but containing in total less than 1% by weight glucose, fructose and sucrose), and RAFTILINE^{®}.RTM. HP (with a (DP) of at least 23, commonly with a (DP) of about 25, and virtually free of glucose, fructose and sucrose). Inutec^{®}N25, a non-derivatised inulin with a DP of 25, is available from Orafti.

Inulins with a lower degree of polymerisation, usually defined as a (DP) <10, are commonly named inulo-oligosaccharides, fructo-oligosaccharides or oligofructose. Oligofructose can be conventionally obtained by partial (preferably enzymatic) hydrolysis of inulin and can also be obtained by enzymatic in vitro synthesis from sucrose according to techniques which are well-known in the art. Several grades of oligofructose are commercially available, for example as RAFTILOSE^{®} from Orafti, (Tienen, Belgium), e.g. RAFTILOSE^{®} P95 with a mean content of about 95% by weight of oligofructose with a degree of polymerisation (DP) ranging from 2 to 7 and containing about 5% by weight in total of glucose, fructose and sucrose.

Various inulin derivatives and methods for the preparation of inulin derivatives are described in US 6,534,647 (Stevens et al).

Inulins derivatised with hydrophobic alkyl chains on the polyfructose backbone are commercially available, for example Inutec^{®} SP1 (SP1) from Orafti (Tienen, Belgium).

Starch is a well-known carbohydrate that is abundantly present in many plants as a biodegradable reserve polysaccharide. Starch molecules are polymers composed of D-glucosyl units which are linked to one another by α-1,4 glucosyl-glucosyl bonds, thus forming a linear chain starch structure (termed amylose) or by α-1,4 and α-1,6 glucosyl bonds thus forming a branched chain starch structure (termed amylopectin) having a α-1,6 glucosyl-glucosyl bond at the branching point. Starch occurs in nature as a polydisperse mixture of polymeric molecules which have, depending on the plant source, mainly a linear structure or mainly a branched structure. Starch can also occur in nature as a polydisperse mixture of molecules with said structures. The degree of polymerisation (DP), i. e. the number of glucosyl units linked to one another in a starch molecule, may widely vary and it largely depends on the plant source and the harvesting time.

The linkages between the glucosyl units are sensitive to hydrolysis, heat and shearing forces. This phenomenon is industrially exploited to prepare various starch derivatives, generically termed herein starch hydrolysates, through acidic hydrolysis, enzymatic hydrolysis, thermal treatment or shearing, or through combinations of said treatments. Depending on the source of the starch, the hydrolysis catalyst, the hydrolysis conditions, the thermal treatment and/or the shearing conditions, a wide variety of starch hydrolysates can be obtained, ranging from a product essentially composed of glucose, over products commonly termed glucose syrups, to products commonly termed maltodextrins and dextrins. Starch hydrolysates are well known in the art.

D-glucose (dextrose) presents strong reducing power. Starch hydrolysates are polydisperse mixtures, composed of D-glucose, oligomeric (DP < 10) and/or polymeric (DP > 10) molecules composed of D-glucosyl chains, which also present reducing power resulting from the presence of D-glucose and reducing sugar units (which are essentially terminal glucosyl units) on the oligomeric and polymeric molecules.

As a result, starting from a given starch product, the greater the extent of the hydrolysis, the more molecules (monomeric D-glucose, oligomeric and remaining polymeric molecules) will be present in the hydrolysate, and thus the higher the reducing powder of the starch hydrolysate obtained. Accordingly, the reducing power of starch hydrolysates has become the distinguishing feature of choice to differentiate and designate the various starch hydrolysate products. The reducing power is expressed as dextrose equivalents (D. E.) which formally corresponds to the grams of D-glucose (dextrose) per 100 grams of dry substance. D-glucose having per definition a D. E. of 100, the D. E. indicates the amount of D-glucose and reducing sugar units (expressed as dextrose) in a given product on a dry product basis. Thus the D. E. is in fact also a measurement of the extent of hydrolysis of the starch and also a relative indication of the average molecular weight of the glucose polymers in the starch hydrolysate.

The D. E. of starch hydrolysates, apart from hydrolysates composed essentially of D-glucose, may range from 1 to about 96 and starch hydrolysates are commercially available in a wide variety of grades based on the D. E.

Hydrolysates with a D. E. greater than 20 are commonly termed glucose syrups. Glucose syrups with a D. E. up to 47 can be dried by conventional techniques, for example by spray drying, to yield so-called "dried glucose syrups" in powder form, containing a maximum of about 5 wt% humidity.

Hydrolysates with a D. E. of 20 or less are commonly termed maltodextrins and dextrins. The manufacturing process usually involves a spray drying step at the end, yielding these hydrolysate products in powder form also containing a maximum of about 5 wt% humidity (wt% indicates % by weight).

Glucose syrups, maltodextrins and dextrins are made industrially at large scale from various starch sources under controlled hydrolysis conditions according to well-known methods. The various grades of starch hydrolysates obtained are usually defined by their starch source material and by their D. E. value, often in combination with an indication of the method of manufacture (e.g. maltodextrins/dextrins).

Although following certain Regulations the term "maltodextrins" is reserved to designate products derived from corn starch, the term maltodextrin (s) used herein is not limited to hydrolysates of corn starch, but indicates herein starch hydrolysates with a D. E. of 20 or less obtained from starch from any source.

Typical commercial sources of starch are corn, potato, tapioca, rice, sorgum and wheat. However, the starch hydrolysates suitable for use in connection with the present invention are not limited to starch from said sources, they extend to starch hydrolysates obtained from starch from any source.

Glucose syrups, maltodextrins and dextrins are well known and commercially available. For example, the production, properties and applications of glucose syrups and maltodextrins have been described in review articles in the book Starch Hydrolysis Products, Worldwide Technology, Production and Applications, Weinheim VCH Publishers Inc. (1992). Furthermore, in the technical brochure "GLUCIDEX Brochure 8/ 09.98" from the company Roquette, describes maltodextrins and dried glucose syrups are described and various grades are offered for sale.

Controlling the stability of proteins during processing and on storage is recognised as a problem in many industries, in particular the pharmaceutical and biotechnology industries. Difficulties encountered with proteins may make manufacture of proteins difficult, result in low yields and render processes uneconomic. Proteins are exposed to conditions during processing that may result in degradation of the protein and loss of function, thus there is a need to improve methods of protein processing to protect the protein and minimise damage or loss of protein. Additionally there is a need to improve protein solubility and reduce protein aggregation. There is also a desire to improve methods that involve processing of proteins in solution, so that processing can be performed at higher protein concentration, thereby reducing volumes. Unfortunately, aggregation is generally promoted at high protein concentration and regions of high local protein concentration are hard to eliminate in large reaction vessels. Detergents or chaotropes can be used to maintain protein solubility, but detergents are extremely difficult to remove during downstream processing, and there are additional drawbacks with the chemicals used, for example the denaturant guanidine hydrochloride is toxic and corrodes the stainless steel vessels and pipes of protein production equipment. Long exposure of a protein to the denaturant urea may result in modification of amino acids, e.g. carbamylation. Detergents and solubilising agents such as arginine are incompatible with several of the most common analytical techniques used in protein laboratories (e.g. Bradford Assay, SDS-PAGE gels, RP-HPLC). Buffer exchange to remove such contaminants is possible, but results in an unknown dilution or loss of the sample and may cause protein aggregation.

Problems addressed by the present invention include stabilisation of proteins against conformational change (e.g. maintaining protein in a denatured conformation, or native conformation), thereby reducing aggregation of proteins, improving protein solubility and protecting proteins against degradation. The present invention also seeks to improve solubility of proteins so that they are maintained in solution, in a denatured or native non-aggregated form, with reduced adherence to the vessel or other protein molecules, and reduced propensity to aggregate.

It is an object of the present invention to provide methods for stabilisation, protection and solubilisation of proteins, that can be used in methods of protein processing, in particular in methods involving protein folding, purification, concentration and/or storage such that the disadvantages associated with present methods are alleviated.

### Disclosure of Invention

In a first aspect, the present invention provides a method of stabilising a protein, characterised by contacting the protein with a sugar polymer derivative, said sugar polymer derivative not being an α-, β- or γ-cyclodextrin, wherein the sugar polymer derivative is an inulin derivative derivatised by one or more type(s) of non-polar hydrocarbyl group selected from the group consisting of a linear alkyl derivative(s) and a branched alkyl derivative(s) and wherein the protein is stabilised against aggregation, conformational change and/or degradation.

In preferred stabilising methods, the protein and sugar polymer derivative are contacted in aqueous solution. A stabilising method may further involve desiccation, dehydration, evaporation, or lyophilisation (freeze drying) of the aqueous solution.

In stabilising methods of the invention the protein is stabilised against conformational change. The protein can be stabilised in a denatured form, which can be unfolded or partially folded, or can be stabilised in native conformation. In methods involving protein in native conformation, the protein is stabilised against denaturation.

In stabilising methods of the invention the protein is stabilised against aggregation. Stabilising methods of the invention are also useful to protect protein against degradation.

We also describe herein, a method of solubilising a protein, characterised by contacting the protein with a sugar polymer derivative, said sugar polymer derivative not being an α- β- or γ-cyclodextrin.

Stabilising and solubilising methods according to the invention and described herein are useful in methods of protein processing, in particular those which comprise or consist of a method of folding protein. Stabilising and solubilising methods can be employed in protein processing methods that comprise one or more protein purification step(s), e.g. dialysis, diafiltration, ultrafiltration and/or chromatography.

According to the invention, a sugar polymer derivative is used to stabilise protein against conformational changes, such as denaturation of native protein or renaturation of denatured (unfolded or partially folded) protein, helping to maintain the protein in the desired configuration. A sugar polymer derivative will act to stabilise proteins against degradation, e.g. by heat, electromagnetic radiation, shear stress, proteolysis, or by chemical modification such as reduction, oxidation, or carbamylation. In methods of the invention, a sugar polymer derivative can be used to stabilise a protein in aqueous solution, or in dry form, e.g. produced by desiccation, dehydration, evaporation or lyophilisation (freeze drying) of an aqueous solution.

We also describe herein the the use of a sugar polymer derivative, said sugar polymer derivative not being an α-, β- or γ-cyclodextrin, to solubilise a protein.

The term "protein" as used herein encompasses proteins, peptides, polypeptides and oligopeptides. Proteins may be synthetic or naturally occurring, and may be obtained by chemical synthesis, or by recombinant or non-recombinant methods. The protein may be produced using DNA recombination or mutation techniques. The protein may be produced *in vivo* in a whole animal, or in a eukaryotic or prokaryotic cell; alternatively, the protein may be generated using an *in vitro* method such as cell-free *in vitro* translation e.g. using *E.coli* lysate, wheat germ extract, or rabbit reticulocyte. Cell free *in vitro* translation methods can be employed following *in vitro* transcription, e.g. following phage or ribosome display.

A denatured protein can be fully denatured, or partially denatured or renatured such that the protein is in non-native form as unfolded protein and/or partially folded refolding intermediate(s). An aqueous solution or dried sample comprising denatured protein may contain one or more of these forms. A native protein is in a folded, functional conformation. Some protein may also be present in aqueous solution, or in a dried sample, in the form of contaminating aggregates and/or inclusion bodies.

Sugar polymers derivatives suitable for use in methods of the invention and described herein are those which, at an appropriate concentration and in appropriate conditions, can maintain a denatured protein in solution in a non-native, unfolded or partially-folded soluble state. The term "sugar polymer derivative" excludes α-, β- or γ-cyclodextrin and derivatives thereof. One, or more (i.e. a mixture of), sugar polymer derivative(s) may be employed in methods or uses of the invention. Suitable sugar polymer derivatives are those which are capable of shielding hydrophobic amino acid side chains or modifying the net protein charge or hydrogen bonding characteristics. The term "sugar polymer derivative" as used herein encompasses polymeric and oligomeric saccharide molecules comprising three or more monosaccharide units. The sugar polymer derivative can be a linear or non-linear amphipathic sugar polymer derivative.

Sugar polymer derivatives employed may comprise one or more sugar(s) selected from the group consisting of: erythrose, threose, ribose, arabinose, xylose, lyxose, allose, altrose, glucose, mannose, gulose, idose, galactose, talose, psicose, fructose, sorbose, tagatose, xylulose and ribulose. Of these sugars, glucose, fructose, mannose and/or galactose, are the four most common simple (monomer) sugar units. The sugar polymer derivative can be a dextran, cellulose, amylose, starch, pullulan, mannan, chitin, chitosan, inulin, levan, xylan, cyclodextrin (provided that it is not an alpha, beta or gamma cyclodextrin), cycloamylose or a derivative thereof. Suitable sugar polymer derivatives are disclosed in US 5,202,433 and 5,204,457..

Amphipathic sugar polymers are capable of hydrophobic interaction with native and/or denatured protein. Derivatisation of the sugar polymer with appropriate substituents enhances the amphipathic nature and strength of interaction with the protein. Suitable substituents for sugar polymer derivatives used in the methods, uses and kits described herein include substituents selected from the group consisting of alkyl groups having from 1 to 25 carbon atoms, alkenyl and alkynyl groups having from 2 to 25 carbon atoms, haloalkyl groups having from 1 to 25 carbon atoms, cycloalkyl groups having from 3 to 9 carbon atoms, aryl groups having from 6 to 14 carbon atoms, aralkyl groups comprising alkyl groups having from 1 to 25 carbon atoms which are substituted with 1 or more aryl groups having from 6 to 14 carbon atoms, fatty acid groups having from 2 to 25 carbon atoms and polyols having from 1 to 25 carbon atoms. One or more substituents per carbohydrate molecule can be present. The alkyl, alkenyl, alkynyl, haloalkyl, aralkyl, fatty acid and polyol groups may be straight chain or branched chain groups. Preferably, the substituents are selected from alkyl, alkenyl and alkynyl groups having from 2 to 25 carbon atoms, more preferably they are selected from alkyl, alkenyl and alkynyl groups having from 3 to 22 carbon atoms, and most preferably they are selected from alkyl, alkenyl and alkynyl groups having from 3 to 18 carbon atoms.

The sugar polymer derivative can be a cyclic sugar polymer derivative comprising 3 or more saccharide units, which may be a glucosan, such as a cyclodextrin derivative, excluding α-, β-, and γ- cyclodextrins.

When the sugar polymer derivative is a cyclic sugar polymer derivative such as a glucosan, e.g. a cyclodextrin derivative, the concentration used in the methods described herein to stabilise protein is preferably 100 to 10,000 times the molar concentration of protein present, more preferably from 100 to 5,000 times the molar concentration of protein present, yet more preferably from 100 to 2,000 times the molar concentration of protein present, further preferably from 500 to 1,500 times the molar concentration of protein present, most preferably about 1,000 times the molar concentration of protein present.

In methods and uses described herein, the sugar polymer derivative is preferably a linear or branched sugar polymer derivative comprising three or more monosaccharide units, such as a fructosan, e.g. an inulin derivative, or a glucoside such as a glucoside hydrocarbyl derivative. Preferred derivatives include inulin derivatives of formula (I) and glucoside hydrocarbyl derivatives of formula (II) as described herein. A glucoside hydrocarbyl derivative of formula (II) is preferably a hydrocarbyl urethane of a starch hydrolysate, particularly preferably a glucoside hydrocarbyl derivative of formula (IIa) composed of units of formula (III) as defined herein.

Suitably an inulin derivative or glucoside hydrocarbyl has a degree of polymerisation of from about 3 to 500, 3 to 250 or 3 to 100, preferably from 3 to 50, more preferably from 10 to 50, yet more preferably from 15 to 40, further preferably from 20 to 30, e.g. 20, 21, 22, 23, 24, 25, 26, 27, 28, 29, 30. It is preferred that inulin derivatives or glucoside hydrocarbyl derivatives are derivatised by one or more type(s) of non-polar hydrocarbyl group, e.g. those selected from the group consisting of: alkyl groups having from 1 to 25 carbon atoms, alkenyl and alkynyl groups having from 2 to 25 carbon atoms, haloalkyl groups having from 1 to 25 carbon atoms, cycloalkyl groups having from 3 to 9 carbon atoms, aryl groups having from 6 to 14 carbon atoms, aralkyl groups comprising alkyl groups having from 1 to 25 carbon atoms which are substituted with 1 or more aryl groups having from 6 to 14 carbon atoms, fatty acid groups having from 2 to 25 carbon atoms and polyols having from 1 to 25 carbon atoms. Preferably, the one or more non-polar hydrocarbyl group is selected from: alkyl groups having from 1 to 25 carbon atoms, and alkenyl and alkynyl groups having from 2 to 25 carbon atoms, preferably 3 to 22, most preferably 3 to 18 carbon atoms. The or a non-polar hydrocarbyl group can be an alkyl group having from 1 to 25, preferably 3 to 22, most preferably 3 to 18 carbon atoms, and/or the or a non-polar hydrocarbyl group can be an alkenyl or alkynyl group having from 2 to 25, preferably 3 to 22, most preferably 3 to 18 carbon atoms.

Sugar polymer derivatives suitable for the methods and uses described herein typically have an average degree of substitution per saccharide unit of from 0.01 to 3.0 or 0.02 to 3.0, preferably from 0.05 to 1.0, most preferably from 0.05 to 0.5. Preferred inulin derivatives typically have an average degree of substitution per saccharide unit in the range of from 0.01 to 3.0, or 0.02 to 3.0, preferably from 0.02 to 1.0 or 0.05 to 1.0, most preferably from 0.05 to 0.5 or 0.03 to 0.3. Preferred glucoside hydrocarbyl derivatives generally have a degree of substitution in the range of from 0.01 to 2.0, preferably 0.02 to 1.0, more preferaby 0.03 to 0.3.

In a preferred aspect, the invention provides a method of stabilising a protein characterised by contacting the protein with an inulin derivative. Preferably, the protein and inulin derivative are contacted in aqueous solution. Methods of stabilisation of protein in aqueous solution may further comprise desiccation, dehydration, evaporation or lyophilisation (freeze drying) of the aqueous solution to produce a dried protein/inulin derivative composition. Drying is particularly useful to help maintain the protein conformation, integrity (and, if native, function), if the protein is to be stored for a length of time. In such methods the protein is stabilised by the inulin derivative against conformational change. The protein to be stabilised can be in the form of a denatured protein, or in native conformation. Denatured proteins are stabilised against aggregation, native proteins are stabilised against denaturation. Both denatured and native protein are protected against degradation.

IWe also describe herein a method of solubilising a protein, characterised by contacting the protein with an inulin derivative. Also provided is a method for stabilising protein in non-native configuration comprising providing to an aqueous solution of protein in non-native conformation, an inulin derivative at a concentration sufficient to inhibit protein folding. The invention further provides a method for stabilising protein in native conformation comprising providing to an aqueous solution of protein in native conformation an inulin derivative at a concentration sufficient to maintain the protein in native conformation.

A concentration of sugar polymer derivative, e.g. inulin derivative, sufficient to inhibit protein folding is a concentration at which renaturation of denatured protein is retarded. At appropriate concentrations, there is less return of structure than would occur if the sugar polymer derivative were not present, because the sugar polymer derivative helps to maintain the protein in non-native configuration. The presence of the sugar polymer derivative reduces the rate of refolding compared to that which would be observed If the derivative was not present. The concentration of inulin derivative for stabilising a given protein in a given conformation (denatured or native) will depend on various factors such as the temperature, solution conditions protein concentration and the protein itself. Suitable concentrations can be determined readily by the skilled person.

The concentration of the inulin derivative for stabilising protein will generally fall within the range of from about 0.1 to about 1000 times the molar concentration of protein, preferably about 0.5 to about 500, about 1 to about 300, about 1 to about 100, about 1 to about 50, about 1 to about 30, about 1 to about 25, or about 1 to about 20 times the molar concentration of protein. Typically, the inulin derivative(s) is present in solution at a concentration of from about 0.002 mg/ml to about 100 mg/ml, preferably about 0.01 mg/ml to about 50 mg/ml, about 0.02 mg/ml to about 10 mg/ml or about 0.25 to 2.5 mg/ml. The upper limit for concentration of an inulin derivative in aqueous solution is governed by the solubility of that inulin derivative in the solution.

Methods of the invention in which an inulin derivative is employed to stabilise and protect protein are useful in methods of protein processing. e.g. in protein folding methods and/or processing involving one or more protein purification step(s), such as dialysis, diafiltration, ultrafiltration and chromatography.

The invention provides the use of an inulin derivative to stabilise a protein against conformational change and/or degradation, and can be used to stabilise a protein in aqueous solution or in dry form.

A suitable inulin derivative for use in methods and uses of the invention is lnutec®SP1 (Orafti, Belgium).

A preferred inulin derivative for a method or use described herein is a compound of formula (I):

G(O-CO-NH-R¹)ₐ-(F(O-CO-NH-R²)_{b}]ₙ (I)

wherein:
G is a terminal glucosyl unit in which one or more hydroxyl groups thereof may be substituted with a group or groups of formula (O-CO-NH-R¹);
R¹ is a hydrocarbyl group selected from the group consisting of alkyl groups, alkenyl groups, alkynyl groups, haloalkyl groups, cycloalkyl groups, aryl groups and aralkyl groups and, where there is more than one (O-CO-NH-R¹) group on the glucosyl unit, each R¹ group may be the same or different;
a is an integer of from 0 to 4;
F is a fructosyl unit in which one or more hydroxyl groups thereof may be substituted with a group or groups of formula (O-CO-NH-R²);
R² is a hydrocarbyl group selected from the group consisting of alkyl groups, alkenyl groups, alkynyl groups, haloalkyl groups, cycloalkyl groups, aryl groups and aralkyl groups and, where there is more than one (O-CO-NH-R²) group on the fructosyl unit, each R² group may be the same or different;
b is an integer of from 0 to 3 and from 0 to 4 for the terminal fructosyl unit;
n is an integer of from 2 to 499 preferably of from 2 to 249, 2 to 99, 2 to 49, 9 to 49, 14 to 39, 19 to 29, or 19 to 24,
each unit of formula F(O-CO-NH-R²)_{b} may be the same or different from any other unit of formula F(O-CO-NH-R²)_{b}; and
the average degree of substitution per glucosyl or fructosyl unit is from 0.01 to 3.0.

Where R¹ or R² is an alkyl group, it is a linear or branched chain alkyl group having from 1 to 25 carbon atoms; preferably, it has from 3 to 22 carbon atoms, and most preferably from 3 to 18 carbon atoms.

Where R¹ or R² is an alkenyl group, it is a linear or branched chain alkenyl group having from 2 to 25 carbon atoms; preferably, it has from 3 to 22 carbon atoms, and most preferably from 3 to 18 carbon atoms.

Where R¹ or R² is an alkynyl group, it is a linear or branched chain alkynyl group having from 2 to 25 carbon atoms; preferably, it has from 3 to 22 carbon atoms, and most preferably from 3 to 18 carbon atoms.

Where R¹ or R² is a haloalkyl group, it is an alkyl group as defined above which is substituted with 1 or more halogen atoms (e.g. fluorine, chlorine or bromine atoms). Preferably, said haloalkyl groups have from 1 to 3 halogen atom substituents, more preferably from 1 to 3 fluorine or chlorine atom substituents. Preferably, said haloalkyl groups have from 3 to 22 carbon atoms, and most preferably from 3 to 18 carbon atoms.

Where R¹ or R² is a cycloalkyl group, it is a cyclic alkyl group having from 3 to 9 carbon atoms; preferably, said cycloalkyl groups have from 3 to 7 carbon atoms and most preferably from 4 to 6 carbon atoms.

Where R¹ or R² is an aryl group, it is an aromatic group having from 6 to 14 carbon atoms in one or more rings, e.g. a phenyl group or a naphthyl group.

Where R¹ or R² is an aralkyl group, it is an alkyl group as defined above that is substituted with one or more aromatic groups having from 6 to 14 carbon atoms in one or more rings, e.g. a benzyl group or a triphenylmethyl group.

Each group R¹ and R² may be selected from alkyl groups having from 1 to 25 carbon atoms, and alkenyl and alkynyl groups having from 2 to 25, preferably 3 to 22, most preferably 3 to 18 carbon atoms. One or more of the groups R¹ and R² can be an alkyl group having from 1 to 25, preferably 3 to 22, most preferably 3 to 18 carbon atoms; suitably one or more of groups R¹ and R² is an alkenyl or alkynyl group having from 2 to 25, preferably 3 to 22, most preferably 3 to 18 carbon atoms. Each alkyl group R¹ and R² can be a linear alkyl group having from 1 to 25, preferably 3 to 22, most preferably 3 to 18 carbons or branched alkyl group having from 3 to 25, preferably 3 to 22, most preferably 3 to 18 carbons.

The average degree of substitution per glucosyl or fructosyl unit is suitably from in the range of from 0.01 to 3.0, or 0.02 to 3.0, preferably from 0.02 to 1.0 or 0.05 to 1.0, most preferably from 0.05 to 0.5 or 0.03 to 0.3. The compound of formula (I) can be a polydisperse linear or slightly branched inulin N-alkylurethane, e.g. selected from the group consisting of inulin N-n-octylcarbamates, inulin N-n-dodecylcarbamates and inulin N-n-octadecylcarbamates.

We further describe herein, a method of stabilising a protein, characterised by contacting the protein with a glucoside derivative, such as a glucoside hydrocarbyl derivative.

In preferred stabilising methods described herein, the protein and glucoside derivative, e.g. glucoside hydrocarbyl derivative are contacted in aqueous solution. A stabilising method may further involve desiccation, dehydration, evaporation, or lyophilisation (freeze drying) of the aqueous solution.

In such stabilising methods the glucoside derivative (e.g. glucoside hydrocarbyl derivative) acts to stabilise the protein against conformational change. The protein can be stabilised in a denatured form, which can be unfolded or partially folded, or can be stabilised in native conformation. In methods involving contacting the glucoside derivative (e.g. glucoside hydrocarbyl derivative) with protein in native conformation, the protein is stabilised against denaturation. In stabilising methods the protein is stabilised against aggregation by the glucoside derivative, which is suitably a glucoside hydrocarbyl derivative. Stabilising methods in which glucoside derivative(s) (e.g. glucoside hydrocarbyl derivative(s)) are used are also useful to protect protein against degradation.

In a yet further aspect, we describe a method of solubilising a protein, characterised by contacting the protein with a glucoside derivative (e.g. glucoside hydrocarbyl derivative).

Stabilising and solubilising methods in which glucoside derivative(s), such as glucoside hydrocarbyl derivative(s), are used are useful in methods of protein processing, in particular those which comprise or consist of a method of folding protein. Such stabilising and solubilising methods can be employed in protein processing methods that comprise one or more protein purification step(s), e.g. dialysis, diafiltration, ultrafiltration and/or chromatography.

In an additional aspect, we describe the use of a glucoside derivative, such as a glucoside hydrocarbyl derivative, to stabilise a protein against aggregation, conformational change and/or degradation.

As described herein, a glucoside derivative, which is preferably a glucoside hydrocarbyl derivative, is used to stabilise protein against conformational changes, such as denaturation of native protein or renaturation of denatured (unfolded or partially folded) protein, helping to maintain the protein in the desired configuration. A glucoside derivative will act to stabilise proteins against degradation, e.g. by heat, electromagnetic radiation, shear stress, proteolysis, or by chemical modification such as reduction, oxidation, or carbamylation. In the methods described herein, a glucoside derivative, glucoside hydrocarbyl derivative, can be used to stabilise a protein in aqueous solution, or in dry form, e.g. produced by desiccation, dehydration, evaporation or lyophilisation (freeze drying) of an aqueous solution.

We also describe the use of a glucoside derivative, suitably a glucoside hydrocarbyl derivative, to solubilise a protein.

The glucoside derivative is preferably a glucoside hydrocarbyl derivative and is further preferably a glucoside hydrocarbyl derivative of formula (II):

[G(O-CO-NH-R¹)ₐ]ₙ (II)

wherein:
G is a glucosyl unit in which one or more hydroxyl groups thereof may be substituted with a group or groups of formula (O-CO-NH-R¹);
R¹ is a hydrocarbyl group selected from the group consisting of alkyl groups, alkenyl groups, alkynyl groups, haloalkyl groups, cycloalkyl groups, aryl groups and aralkyl groups and, where there is more than one (O-CO-NH-R¹) group on each glucosyl unit, each R¹ group may be the same or different;
a is an integer of from 0 to 4 for a terminal glucosyl unit, 0 to 3 for a non-branched, non-terminal glucosyl unit and 0 to 2 for a branched, non-terminal glucosyl unit;
n is an integer of from 3 to 499 preferably of from 3 to 249, 3 to 99, 3 to 49, 9 to 49, 14 to 39, 19 to 29, or 19 to 24,
each unit of formula G(O-CO-NH-R¹)ₐ may be the same or different from any other unit of formula G(O-CO-NH-R¹)ₐ; and
the average degree of substitution per glucosyl unit is from 0.01 to 2.0.

Where R¹ is an alkyl group, it is a linear or branched chain alkyl group having from 1 to 25 carbon atoms; preferably, it has from 3 to 22 carbon atoms, and most preferably from 3 to 18 carbon atoms.

Where R¹ is an alkenyl group, it is a linear or branched chain alkenyl group having from 2 to 25 carbon atoms; preferably, it has from 3 to 22 carbon atoms, and most preferably from 3 to 18 carbon atoms.

Where R¹ is an alkynyl group, it is a linear or branched chain alkynyl group having from 2 to 25 carbon atoms; preferably, it has from 3 to 22 carbon atoms, and most preferably from 3 to 18 carbon atoms.

Where R¹ is a haloalkyl group, it is an alkyl group as defined above which is substituted with 1 or more halogen atoms (e.g. fluorine, chlorine or bromine atoms). Preferably, said haloalkyl groups have from 1 to 3 halogen atom substituents, more preferably from 1 to 3 fluorine or chlorine atom substituents. Preferably, said haloalkyl groups have from 3 to 22 carbon atoms, and most preferably from 3 to 18 carbon atoms.

Where R¹ is a cycloalkyl group, it is a cyclic alkyl group having from 3 to 9 carbon atoms; preferably, said cycloalkyl groups have from 3 to 7 carbon atoms and most preferably from 4 to 6 carbon atoms.

Where R¹ is an aryl group, it is an aromatic group having from 6 to 14 carbon atoms in one or more rings, e.g. a phenyl group or a naphthyl group.

Where R¹ is an aralkyl group, it is an alkyl group as defined above that is substituted with one or more aromatic groups having from 6 to 14 carbon atoms in one or more rings, e.g. a benzyl group or a triphenylmethyl group.

Preferably, one or more of the groups R¹ can be a linear or branched chain alkyl group having from 1 to 25, preferably 3 to 22, most preferably 3 to 18 carbon atoms and/or one or more of groups R¹ can be an alkenyl or alkynyl group having from 2 to 25, preferably 3 to 22, most preferably 3 to 18 carbon atoms. Suitably each alkyl group R¹ is a linear alkyl group having from 1 to 25, preferably 3 to 22, most preferably 3 to 18 carbons or a branched alkyl group having from 3 to 25, preferably 3 to 22, most preferably 3 to 18 carbons.

In a compound of formula (II), the average degree of substitution per glucosyl unit is from 0.01 to 2.0, preferably from 0.02 to 1.0, and most preferably from 0.03 to 0.3.

The compound of formula (II) can be a polydisperse linear or branched glucoside N-hydrocarbyl urethane. Each glucosyl unit G may be a D-glucosyl or L-glucosyl unit, preferably a D-glucosyl unit. The glucosyl units can be linked via 1,4-linkages or 1,6-linkages, and each linkage can be an α-linkage or a β-linkage.

In preferred aspects of the methods and uses described herein, said compounds of formula (II) are hydrocarbyl urethanes of starch hydrolysates.

Particularly preferred hydrocarbyl urethanes of starch hydrolysates are glucoside hydrocarbyl derivatives of formula (IIa) composed of units of formula (III):

G' (O-CO-NH-R¹)_{b} (III)

Wherein
G' represents a glucosyl unit of a starch hydrolysate molecule, the starch hydrolysate having a Dextrose Equivalent (D. E.) ranging from 1 to 47,
R¹ is a hydrocarbyl group as defined above, and
b represents the number of hydrocarbyl carbamate groups per glucosyl unit, which number is commonly expressed as the degree of substitution (DS), i.e. the average number of hydrocarbyl substituents per glucosyl unit of the glucoside hydrocarbyl urethane of formula (IIa), with said DS value ranging from 0.01 to 2.0.

The number of hydroxyl groups per glucosyl unit of the subject glucoside molecules which theoretically can be substituted by a carbamate group is, for a non-terminal, non-branched glucosyl unit, a maximum of 3, whereas the number for a terminal or for a non-terminal branched glucosyl unit is, respectively, 4 or 2. Furthermore, since the DS represents an average number of substituents per glucosyl unit, it is evident that in a glucoside N-hydrocarbyl carbamate (IIa) molecule there may be glucosyl units present which are not substituted by a hydrocarbyl carbamate group (thus b in formula (III) being zero for said glucosyl unit).

Starch hydrolysates commonly appear in the form of a polydisperse mixture of glucoside molecules. Accordingly, when such a mixture is used, as is usually the case, as starting material for the preparation of a glucoside hydrocarbyl urethane (IIa), the product obtained is also a corresponding polydisperse mixture of glucoside hydrocarbyl urethanes (IIa). Such polydisperse mixtures constitute a preferred embodiment of the glucoside hydrocarbyl urethanes (IIa) for utilisation in the methods and uses described herein.

Commercial grades of starch hydrolysates, composed of said polydisperse mixture of glucoside molecules and having a D. E. ranging from 1 to 47 are very suitable for the preparation of glucoside hydrocarbyl urethanes (IIa).

Typically suitable starch hydrolysates for use in the preparation of glucoside N-hydrocarbyl urethanes (IIa) are for example GLUCIDEX^{®} maltodextrins and GLUCIDEX^{®} dried glucose syrups which are available from ROQUETTE company, such as the maltodextrins of type 1 (potato based with D. E. max 5), type 2 (Waxy Maize based with D. E. max 5), type 6 (Waxy Maize based with D. E. 5 to 8), type 9 (Potato based with D. E. 8 to 10), and maltodextrins of type 12 (D. E. 11 to 14), type 17 (D. E. 15 to 18) and type 19 (D. E. 18 to 20), as well as dried glucose syrups of type 21 (D. E. 20 to 23), type 28E (D. E. 28 to 31), type 29 (D. E. 28 to 31), type 32 (D. E. 31 to 34), type 33 (D. E. 31 to 34), type 38 (D. E. 36 to 40), type 39 (D. E. 38 to 41), type 40 (D. E. 38 to 42) and type 47 (D. E. 43 to 47).

The hydrocarbyl group of the hydrocarbyl urethanes (IIa), i.e. the R¹ group in formula (III) defined herein above, is preferably a saturated C₃-C₂₂ alkyl group, more preferably a saturated C₄-C₁₈ alkyl group, even more preferably a saturated linear C₄-C₁₈ alkyl group, most preferably a saturated linear C₆-C₁₈ alkyl group. Typically suitable alkyl groups include butyl, hexyl, octyl, decyl, dodecyl, tetradecyl, hexadecyl and octadecyl groups.

In another aspect, the hydrocarbyl group is a C₃-C₂₂ alkenyl group, preferably a C₄-C₁₈ alkenyl group, most preferably a linear C₆-C₁₈ alkenyl group.

Typically suitable alkenyl groups include butenyl, hexenyl, octenyl, decenyl, dodecenyl, tetradecenyl, hexadecenyl and octadecenyl groups.

In the urethane (IIa), all R¹ groups of the units of formula (III) may be the same or different. The latter urethanes (IIa) can be easily prepared, according to the method described below, by reacting a starch hydrolysate with an isocyanate of formula R¹-NCO which is in fact a mixture of two or more isocyanates bearing different R¹ groups defined above.

Saturated alkyl isocyanates can be prepared conventionally, e.g. by reacting a primary or secondary alkyl-amine with phosgene. Unsaturated alkylisocyanates can be prepared similarly from alkenyl-amines. α,β-unsaturated alkylisocyanates of formula R²R³C=CH-NCO (IV) wherein the radical R²R³C=CH- corresponds to the group R¹ of formula (III) and wherein R² represents hydrogen or an alkyl group and R³ represents an alkyl or vinyl group, can be prepared by condensation of the aldehyde R²R³CH-CHO with Me₃C-NH2, followed by reaction of the resultant Schiff base (in equilibrium with its enamine form) with phosgene, and thermal elimination of Me₃C-Cl as disclosed by K. Koenig et al. (Angew. Chem., 21 (4), 334-335 (1979)). Furthermore, various unsaturated alkylisocyanates are disclosed, *inter alia* in US 3, 890, 383 and US 3, 803, 062 of Dow Chemical Co. Many alkyl cyanates of formula R¹-N=C=O (R¹ as defined above) are commercially available.

Glucoside hydrocarbyl urethanes (IIa) described herein have a degree of substitution (DS) per glucosyl unit of formula (III) ranging from 0.01 to 2.0, preferably from 0.02 to 1.0, and most preferably from 0.03 to 0.3.

The hydrocarbyl carbamate substituent or substituents can be located at various positions on the glucosyl units of the glucoside hydrocarbyl urethanes (IIa).

The glucoside hydrocarbyl urethanes (IIa) can be prepared in analogy with conventional methods for the preparation of urethanes of monosaccharides, disaccharides, and polysaccharides, for example, by reacting the starch hydrolysate with the selected hydrocarbyl isocyanate or mixture of hydrocarbyl isocyanates, in solution in a solvent which is inert with respect to the starch hydrolysate, the isocyanate and the reaction product.

Suitable solvents include solvents or solvent mixtures which are free of reactive hydroxyl and amine groups, such as for example dimethyl formamide (DMF), dimethyl sulfoxide (DMSO) and N-methyl pyrrolidone (NMP).

The reaction between the starch hydrolysate and the hydrocarbylisocyanate is preferably performed under anhydrous conditions. In view of this, the starch hydrolysate as well as the solvent(s) are dried, preferably to a water content of less than 0.5 wt%, prior to bringing them into contact with the hydrocarbyl isocyanate. Drying can be carried out by conventional techniques, including, for example by heating the starch hydrolysate in a stream of dry air, or by heating the starch hydrolysate under reduced pressure, or by removing the water through azeotropic distillation, optionally under reduced pressure, from a solution of the starch hydrolysate in the solvent chosen for the reaction. During drying a maximum temperature, depending on the nature of the starch hydrolysate and the solvent, should not be exceeded in order to avoid any decomposition or side reaction. Preferably said temperature should be kept below about 80°C.

The reaction is typically carried out by bringing the starch hydrolysate dissolved in a suitable solvent into contact, under gentle to vigorous stirring, with the hydrocarbyl isocyanate in neat form or dissolved in an anhydrous solvent. The reaction can be carried out over a wide temperature range, typically from room temperature up to about 80°C, or the reflux temperature of the reaction mixture if it is lower, preferably at a temperature between about 60 °C and about 80°C.

Typically, the starch hydrolysate is dissolved in a suitable solvent, where necessary under heating. Accordingly, the hydrocarbyl isocyanate (optionally dissolved in the same or in another inert solvent, but which is preferably miscible with the former solvent) is added slowly under stirring to the dissolved glucoside. The desired degree of substitution of the glucoside hydrocarbyl urethane (IIa) can be obtained by controlling the ratio of the reactants. Since the reaction of an hydrocarbyl isocyanate with an alcoholic hydroxyl group to form an urethane is a quantitative reaction, the degree of substitution of the urethane (IIa) can be controlled by the selection of the proper molar ratio of the hydrocarbyl isocyanate per glucosyl unit of the starch hydrolysate. Usually the reaction mixture is heated with stirring for a time period, usually from about 30 minutes to about 24 hours, in order to complete the reaction between the reagents. The reaction mixture is then worked up by conventional techniques, for example, by precipitating the formed urethane (IIa) through pouring the reaction mixture, usually after cooling to room temperature, in a precipitant solvent, which is a solvent that is miscible with the solvent or solvents used to dissolve the reagents but in which the glucoside alkyl urethane (IIa) is not or very poorly soluble. The urethane (IIa) is then physically isolated from the reaction mixture, for example by filtration or centrifugation, washed with a suitable solvent in which the urethane (IIa) is not or only very slightly soluble, and dried using any common technique.

A further convenient method to synthesise a desired urethane (IIa), can be performed in an analogous manner to the one described by W. Gerhardt, Abh. Dtsch. Akad. Wiss. Berlin, KL. Chem. Geol. Biol., Vol 1966 (6), 24-36, (1967) (C. A.,6S, 14323). It involves the transformation in a one-pot reaction in dimethyl formamide of the starch hydrolysate with potassium cyanate and with a selected hydrocarbyl halogenide, preferably an hydrocarbyl bromide.

Methods and uses of sugar polymer derivatives, in particular inulin derivatives and glucoside hydrocarbyl derivatives as described herein can be beneficially applied to stabilisation, protection and solubilisation of proteins which are to be used in foodstuffs, nutritional supplements, cosmetics or pharmaceutical preparations. In this context non-toxic sugar polymer derivatives are usually used, particularly if an appreciable amount of the sugar polymer derivative is to be present in the protein product. If the presence of a significant concentration of the sugar polymer derivative is not desired in the protein product, then the sugar polymer derivative can be readily separated from protein.

Methods and uses of the invention are useful as the sugar polymer derivatives employed influence protein analytical techniques to a much lesser extent than traditional denaturants, solubilisers and protectants e.g. detergents, arginine and guanidine hydrochloride.

Sugar polymer derivatives are generally less expensive than chaperone proteins, so the invention provides a cost effective means of maintaining or improving solubility, particularly in scale-up of processes, where prior art methods may prove uneconomic.

In methods of the invention, aggregation is suppressed without compromising downstream applications of the protein.

Methods of the invention are suitable to enhance protein stability on storage, improve chromatographic purifications, minimise losses during protein concentration and reduce protein aggregation during dialysis. The invention can be applied in all protein processing areas, to increase yields and decrease solution volumes, meaning that capital expenditure is reduced and the efficiency of production processes can be improved.

Kits are provided for performing methods of the invention, kits will generally contain a supply of sugar polymer derivative, e.g. one or more inulin derivative(s) and/or one or more glucoside hydrocarbyl derivative(s) as described herein, preferably one or more inulin derivative(s) of formula (I) and/or one or more glucoside hydrocarbyl derivative(s) of formula (II), optionally together with additional reagents, consumables such as vials, pipette tips and spun columns, and/or instructions for use of the kit.

The kit may also contain buffers and other reagents suitable for use in performing a method of the invention.

The invention also provides a composition comprising a protein, and an inulin derivative with a degree of polymerisation of from about 3 to 500, 3 to 250 or 3 to 100, preferably from 3 to 50, more preferably from 10 to 50, yet more preferably from 15 to 40, further preferably from 20 to 30, e.g. 20, 21, 22, 23, 24, 25, 26, 27, 28, 29, or 30. In a composition of the invention, the inulin derivative may be Inutec^{®}SP1. Inulin derivatives useful in such compositions include those derivatised by one or more type(s) of non-polar hydrocarbyl group selected from the group consisting of: the group consisting of: alkyl groups having from 1 to 25 carbon atoms, alkenyl and alkynyl groups having from 2 to 25 carbon atoms, haloalkyl groups having from 1 to 25 carbon atoms, cycloalkyl groups having from 3 to 9 carbon atoms, aryl groups having from 6 to 14 carbon atoms, aralkyl groups comprising alkyl groups having from 1 to 25 carbon atoms which are substituted with 1 or more aryl groups having from 6 to 14 carbon atoms, fatty acid groups having from 2 to 25 carbon atoms and polyols having from 1 to 25 carbon atoms.

In such compositions the inulin derivative can be a compound of formula (I), thus the invention provides a composition comprising a protein, and an inulin derivative of formula (I):

G(O-CO-NH-R¹)ₐ-[F(O-CO-NH-R²)_{b}]ₙ (I)

wherein:
G is a terminal glucosyl unit in which one or more hydroxyl groups thereof may be substituted with a group or groups of formula (O-CO-NH-R¹);
R¹ is a straight or branched chain saturated or unsaturated hydrocarbyl group having from 1 to 25 carbon atoms and, where there is more than one (O-CO-NH-R¹) group on the glucosyl unit, each R¹ group may be the same or different;
a is an integer of from 0 to 4;
F is a fructosyl unit in which one or more hydroxyl groups thereof may be substituted with a group or groups of formula (O-CO-NH-R²);
R² is a straight or branched chain saturated or unsaturated hydrocarbyl group having from 1 to 25 carbon atoms and, where there is more than one (O-CO-NH-R²) group on the fructosyl unit, each R² group may be the same or different;
b is an integer of from 0 to 3 and from 0 to 4 for the terminal fructosyl unit;
n is an integer of from 2 to 499 preferably of from 2 to 249, 2 to 99, 2 to 49, 9 to 49, 14 to 39, 19 to 29, or 19 to 24,
each unit of formula F(O-CO-NH-R²)_{b} may be the same or different from any other unit of formula F(O-CO-NH-R²)_{b}; and
the average degree of substitution per glucosyl or fructosyl unit is from 0.01 to 3.0.

In a composition according to this aspect of the invention the compound of formula (I) can be a polydisperse linear or slightly branched inulin N-alkylurethane, and may be selected from the group consisting of inulin N-n-octyl-carbamates, inulin N-n-dodecylcarbamates and inulin N-n-octadecylcarbamates.

A further composition described herein may comprise a protein, and a glucoside hydrocarbyl derivative of formula (II):

[G(O-CO-NH-R¹)ₐ]ₙ (II)

wherein:
G is a glucosyl unit in which one or more hydroxyl groups thereof may be substituted with a group or groups of formula (O-CO-NH-R¹);
R¹ is a hydrocarbyl group selected from the group consisting of alkyl groups, alkenyl groups, alkynyl groups, haloalkyl groups, cycloalkyl groups, aryl groups and aralkyl groups and, where there is more than one (O-CO-NH-R¹) group on each glucosyl unit, each R¹ group may be the same or different;
a is an integer of from 0 to 4 for a terminal glucosyl unit, 0 to 3 for a non-branched, non-terminal glucosyl unit and 0 to 2 for a branched, non-terminal glucosyl unit;
n is an integer of from 3 to 499 preferably of from 3 to 249, 3 to 99, 3 to 49, 9 to 49, 14 to 39, 19 to 29, or 19 to 24,
each unit of formula G(O-CO-NH-R¹)ₐ may be the same or different from any other unit of formula G(O-CO-NH-R¹)ₐ; and
the average degree of substitution per glucosyl unit is from 0.01 to 2.0.

In compositions of the invention, the molar concentration of the inulin derivative or glucoside hydrocarbyl derivative is generally in the range of from about 0.1 to about 1000 times the molar concentration of protein, preferably about 0.5 to about 500, about 1 to about 300, about 1 to about 100, about 1 to about 50, about 1 to about 30, about 1 to about 25, or about 1 to 20 times the molar concentration of protein. Compositions can be in liquid, e.g. aqueous form, or can be in dried form. In a preferred embodiment the composition is a pharmaceutical composition, e.g. in aqueous form, which may be suitable for administration by injection, ingestion, inhalation or topical application, or in dried form for reconstitution in a suitable liquid (such as sterile distilled water, saline, or a pharmaceutically acceptable buffer) for administration, e.g. by injection, ingestion, inhalation or topical administration.

Compositions according to the invention, are suitable for use in the diagnosis, treatment, prophylaxis or monitoring of disease in humans and/or animals. Pharmaceutical compositions according to the invention may comprise, for example, therapeutic proteins such as insulin, erythropoietin, antibodies or antibody fragments. Pharmaceutical compositions may contain antigenic proteins for immunisation.

### List of Figures

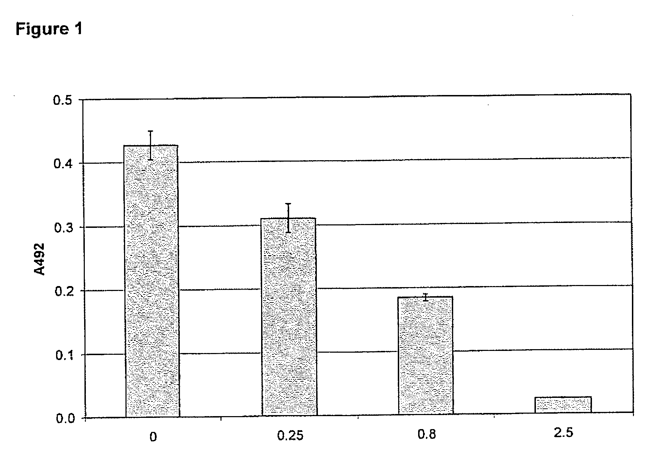

**Figure 1** shows protein aggregation as a function of alkyl-inulin concentration in the solution used to dilute the denatured protein. The final lysozyme concentration was 0.75 mg /ml. The y-axis shows the A492 measurement (aggregation) and the x-axis indicates the alkyl-inulin concentration in mg/ml.

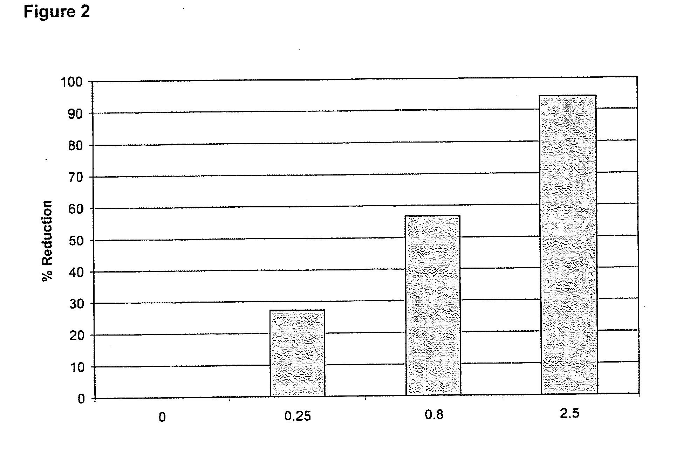

**Figure 2** shows the relative reduction in protein aggregation as a function of alkyl-inulin concentration in the solution used to dilute the denatured protein. The final lysozyme concentration was 0.75 mg /ml. The y-axis shows the percentage reduction in aggregation and the x-axis indicates the alkyl-inulin concentration in mg/ml.

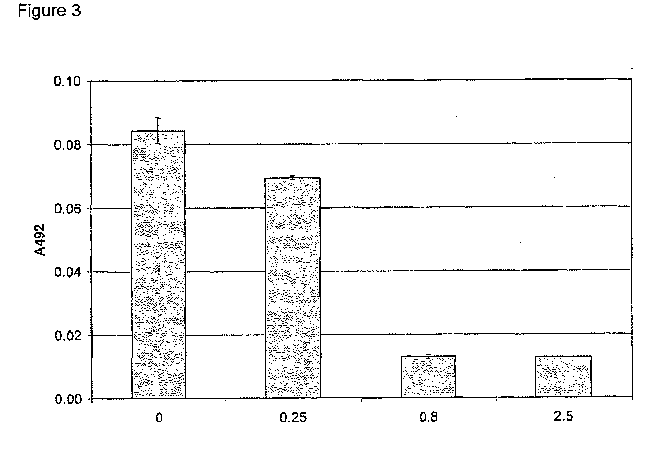

**Figure 3** shows protein aggregation as a function of alkyl-inulin concentration in the solution used to dilute the denatured protein. The final citrate synthase concentration is 0.25 mg/ml. The y-axis shows the A492 measurement (aggregation) and the x-axis indicates the alkyl-inulin concentration in mg/ml.

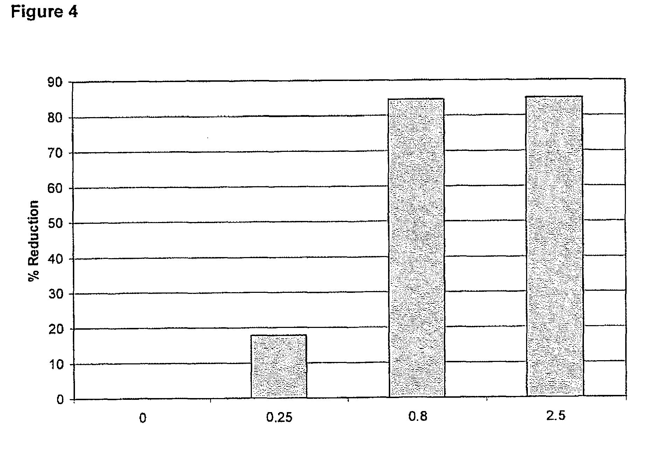

**Figure 4** shows the relative reduction in protein aggregation as a function of alkyl-inulin concentration in the solution used to dilute the denatured protein. The final citrate synthase concentration was 0.25 mg /ml. The y-axis shows the percentage reduction in aggregation and the x-axis indicates the alkyl-inulin concentration in mg/ml.

### Examples

### Example 1 Stabilisation and protection of protein, aggregation suppression during dilution of denatured lysozyme

The test protein, hen egg white lysozyme (Fluka, #62971), was dissolved at a concentration of 15 mg/ml in denaturation buffer, 8 M Urea, 0.1 M Tris-HCl, 32 mM DTT at pH 8.2. The protein was denatured overnight at 4°C. The denatured protein was then diluted 20 times into solutions of 0.1 mM Tris-HCl, 5 mM GSSG at pH 8.2 containing various concentrations of Inutec^{®} SP1 (alkyl-inulin). Aggregation was monitored by measuring the solution turbidity through adsorption of light at 492 nm (A492) after 15 min incubation at room temperature. The results are shown in figures 1 and 2.

### Example 2 Stabilisation and protection of protein, aggregation suppression during dilution of denatured citrate synthase

The test protein, porcine heart citrate synthase (Sigma, #C3260), was dissolved in denaturation buffer, 8 M Urea, 0.1 M Tris-HCl, 32 mM DTT at pH 8.2. The solution was prepared to give a final concentration of 5 mg/ml protein using an ultrafiltration device (Vivaspin 500 PES 10 kDa MWCO, Fisher, #FDP875010X) to perform a buffer exchange into the denaturation buffer. The protein was denatured overnight at 4°C. The denatured protein was then diluted 20 times into solutions of 0.1 mM Tris-HCl, 5 mM GSSG at pH 8.2 containing various concentrations of alkyl-inulin. Aggregation was monitored by measuring the solution turbidity thorough adsorption of light at 492 nm (A492) after 15 min incubation at room temperature. The results are shown in figures 3 and 4.

## Claims

1. A method of stabilising a protein, **characterised by** contacting the protein with a sugar polymer derivative, said sugar polymer derivative not being an α-, β- or γ-cyclodextrin, wherein the sugar polymer derivative is an inulin derivative derivatised by one or more type(s) of non-polar hydrocarbyl group selected from the group consisting of a linear alkyl derivative(s) and a branched alkyl derivative(s) and wherein the protein is stabilised against aggregation, conformational change and/or degradation.

2. A method according to claim 1, **characterised in that** the protein and inulin derivative are contacted in aqueous solution.

3. A method according to claim 2, further comprising drying the solution by desiccation, dehydration, evaporation or lyophilisation.

4. A method according to any one of claims 1 to 3, wherein the protein is stabilised against conformational change.

5. A method according to any one of claims 1 to 4 wherein the protein is a denatured protein.

6. A method according to any one of claims 1 to 4, wherein the protein is in native conformation.

7. A method according to claim 6, wherein the protein is stabilised against denaturation.

8. A method according to any one of claims 1 to 7, wherein the protein is stabilised against aggregation.

9. A method according to any one of claims 1 to 8 wherein the protein is protected against degradation.

10. A method according to any one of claims 1 to 9, wherein the concentration of the inulin derivative is in the range of from 0.1 to 1000 times the molar concentration of protein.

11. A method according to any one of claims 1 to 10, wherein the inulin derivative is present at a concentration of from 0.002 mg/ml to 100 mg/ml.

12. A method of protein processing comprising a method according to any one of claims 1 to 11.

13. A method according to claim 12, comprising a method of folding protein and/or comprising one or more protein purification step(s).

14. A method according to claim 13, wherein said protein purification step(s) is/are selected from the group consisting of: dialysis, diafiltration, ultrafiltration and chromatography.

15. The use of an inulin derivative, which is derivatised by one or more type(s) of non-polar hydrocarbyl group selected from the group consisting of a linear alkyl derivative(s) and a branched alkyl derivative(s), to stabilise a protein against conformational change and/or degradation.

16. A use according to claim 15, to stabilise a protein in aqueous solution.

17. A use according to claim 15 or 16, to stabilise a protein in dry form.

18. A method or use according to any one of claims 1 to 17, wherein the inulin derivative has a degree of polymerisation of from 3 to 500, 3 to 250 or 3 to 100, preferably from 3 to 50, more preferably from 10 to 50, yet more preferably from 15 to 40, further preferably from 20 to 30.

19. A method or use according to any one of claims 1 to 18, wherein the inulin derivative is a compound of formula (I):
G(O-CO-NH-R¹)ₐ-[F(O-CO-NH-R²)_{b}]ₙ (I)
wherein:
G is a terminal glucosyl unit in which one or more hydroxyl groups thereof may be substituted with a group or groups of formula (O-CO-NH-R¹);
R¹ is selected from the group comprising alkyl groups and, where there is more than one (O-CO-NH-R¹) group on the glucosyl unit, each R¹ group may be the same or different;
a is an integer of from 0 to 4;
F is a fructosyl unit in which one or more hydroxyl groups thereof may be substituted with a group or groups of formula (O-CO-NH-R²);
R² is selected from the group comprising alkyl groups and, where there is more than one (O-CO-NH-R²) group on the fructosyl unit, each R² group may be the same or different;
b is an integer of from 0 to 3 and from 0 to 4 for the terminal fructosyl unit;
n is an integer of from 2 to 499 preferably of from 2 to 249, 2 to 99, 2 to 49, 9 to 49, 14 to 39, 19 to 29, or 19 to 24,
each unit of formula F(O-CO-NH-R²)_{b} may be the same or different from any other unit of formula F(O-CO-NH-R²)_{b}; and
the average degree of substitution per saccharide unit is from 0.02 to 3.0.

20. A method or use according to claim 19, wherein each group R¹ and R² is selected from alkyl groups having from 1 to 25 carbon atoms.

21. A method or use according to claim 19, wherein each alkyl group R¹ and R² is a linear alkyl group having from 1 to 25 carbon atoms or a branched alkyl group having from 3 to 25 carbon atoms.

22. A method or use according to any one of claims 19 to 21, wherein the compound of formula (I) is a polydisperse linear or slightly branched inulin N-alkylurethane.

23. A method or use according to any one of claims 19 to 22, wherein the compound of formula (I) is selected from the group consisting of inulin N-n-octyl-carbamates, inulin N-n-dodecylcarbamates and inulin N-n-octadecylcarbamates.

24. A composition comprising a protein and an inulin derivative of formula (I):
G(O-CO-NH-R¹)ₐ-[F(O-CO-NH-R²)_{b}]ₙ (I)
wherein:
G is a terminal glucosyl unit in which one or more hydroxyl groups thereof may be substituted with a group or groups of formula (O-CO-NH-R¹);
R¹ is selected from the group comprising alkyl groups and, where there is more than one (O-CO-NH-R¹) group on the glucosyl unit, each R¹ group may be the same or different;
a is an integer of from 0 to 4;
F is a fructosyl unit in which one or more hydroxyl groups thereof may be substituted with a group or groups of formula (O-CO-NH-R²);
R² is selected from the group comprising alkyl groups and, where there is more than one (O-CO-NH-R²) group on the fructosyl unit, each R² group may be the same or different;
b is an integer of from 0 to 3 and from 0 to 4 for the terminal fructosyl unit;
n is an integer of from 2 to 499 preferably of from 2 to 249, 2 to 99, 2 to 49, 9 to 49, 14 to 39, 19 to 29, or 19 to 24,
each unit of formula F(O-CO-NH-R²)_{b} may be the same or different from any other unit of formula F(O-CO-NH-R²)_{b}; and
the average degree of substitution per saccharide unit is from 0.02 to 3.0.

25. A composition according to claim 24, wherein the compound of formula (I) is a polydisperse linear or slightly branched inulin N-alkylurethane.

26. A composition according to claim 24 or claim 25, wherein the compound of formula (I) is selected from the group consisting of inulin N-n-octyl-carbamates, inulin N-n-dodecylcarbamates and inulin N-n-octadecylcarbamates.

27. A composition according to any one of claims 24 to 26, wherein the molar concentration of the inulin derivative is in the range of from 0.1 to 1000 times the molar concentration of protein.

28. A composition according to any one of claims 24 to 27, which is an aqueous solution.

29. A composition according to any one of claims 24 to 28 in dry form.

30. A composition according to any one of claims 24 to 29, which is a pharmaceutical composition.

## Patentansprüche

1. Verfahren zur Stabilisierung eines Proteins, **gekennzeichnet durch** das in Kontakt bringen des Proteins mit einem Zuckerpolymer-Derivat, wobei das Zuckerpolymer-Derivat kein α-, β-oder γ-Cyclodextrin ist, wobei das Zuckerpolymer-Derivat ein Inulin-Derivat ist, welches mit einer oder mehreren Art(en) nicht-polarer Kohlenwasserstoffgruppen derivatisiert ist, ausgewählt aus der Gruppe, welches aus einem linearen Alkyl-Derivat/-Derivaten und einem verzweigten Alkyl-Derivat/- Derivaten besteht, und wobei das Protein gegenüber Aggregation, Konformationsänderung und/oder Abbau stabilisiert ist.

2. Verfahren nach Anspruch 1, **dadurch gekennzeichnet, dass** das Protein und Inulin-Derivat in wässriger Lösung miteinander in Kontakt gebracht werden.

3. Verfahren nach Anspruch 2, welches weiterhin das Trocknen der Lösung durch Desikkation, Dehydratation, Evaporation oder Lyophilisation umfasst

4. Verfahren nach einem der Ansprüche 1 bis 3, wobei das Protein gegenüber Konformationsänderungen stabilisiert wird.

5. Verfahren nach einem der Ansprüche 1 bis 4, wobei das Protein ein denaturiertes Protein ist.

6. Verfahren nach einem der Ansprüche 1 bis 4, wobei das Protein in nativer Konformation ist.

7. Verfahren nach Anspruch 6, wobei das Protein gegenüber Denaturierung stabilisiert wird.

8. Verfahren nach einem der Ansprüche 1 bis 7, wobei das Protein gegenüber Aggregation stabilisiert wird.

9. Verfahren nach einem der Ansprüche 1 bis 8, wobei das Protein gegen Abbau geschützt wird.

10. Verfahren nach einem der Ansprüche 1 bis 9, wobei die Konzentration des Inulin-Derivats im Bereich des 0,1- bis 1000-fachen der molaren Konzentration des Proteins ist.

11. Verfahren nach einem der Ansprüche 1 bis 10, wobei das Inulin-Derivat in einer Konzentration von 0,002 mg/ml bis 100 mg/ml vorliegt.

12. Verfahren zur Protein-Verarbeitung, welches ein Verfahren nach einem der Ansprüche 1 bis 11 umfasst.

13. Verfahren nach Anspruch 12, welches ein Verfahren zum Falten von Protein und/oder einen oder mehrere Protein-Aufreinigungsschritt(e) umfasst.

14. Verfahren nach Anspruch 13, wobei der/die Protein-Aufreinigungsschritt (e) ausgewählt wird/werden, die aus der Gruppe aus Dialyse, Diafiltration, Ultrafiltration und Chromatographie besteht.

15. Verwendung eines Inulin-Derivates, das durch eine oder mehrere Art(en) von nichtpolaren Kohlenwasserstoffgruppen, ausgewählt aus der Gruppe bestehend aus einem linearen Alkyl-Derivat/-Derivaten und einem verzweigten Alkyl-Derivat/-Derivaten, derivatisiert ist, um ein Protein gegenüber Konformationsänderung und/oder Abbau zu stabilisieren.

16. Verwendung nach Anspruch 15, um ein Protein in wässriger Lösung zu stabilisieren.

17. Verwendung nach Anspruch 15 oder 16, um ein Protein in Trockenform zu stabilisieren.

18. Verfahren oder Verwendung nach einem der Ansprüche 1 bis 17, wobei das Inulin-Derivat einen Polymerisationsgrad von 3 bis 500, 3 bis 250 oder 3 bis 100, vorzugsweise von 3 bis 50, besonders bevorzugt von 10 bis 50, noch mehr bevorzugt von 15 bis 40, weiter bevorzugt von 20 bis 30 hat.

19. Verfahren oder Verwendung nach einem der Ansprüche 1 bis 18, wobei das Inulin-Derivat eine Verbindung der Formel (I) ist:
G(O-CO-NH-R¹)ₐ-[F(O-CO-NH-R²)_{b}]ₙ (I),
wobei:
G eine terminale Glucosyl-Einheit ist, bei der eine oder mehrere Hydroxyl-Gruppe(n) durch eine oder mehrere Gruppe(n) der Formel (O-CO-NH-R¹) substituiert sein kann/können;
R¹ ausgewählt ist aus der Gruppe, welche Alkylgruppen umfasst, und, wo mehr als eine (O-CO-NH-R¹)-Crruppe an der Glucosyl-Einheit sind, wobei jede Gruppe R¹ gleich oder verschieden sein kann:
a eine ganze Zahl von 0 bis 4 ist;
F eine Fructosyl-Einheit ist, bei der eine oder mehrere Hydroxyl-Crruppe(n) durch eine oder mehrere Gruppe(n) der Formel (O-CO-NH-R²) substituiert sein kann/können;
R² ausgewählt ist aus der Gruppe, welche Alkylgruppen umfasst, und, wo mehr als eine (O-CO-NH-R²)-Gruppe an der Fructosyl-Einheit sind, jede Gruppe R² gleich oder
verschieden sein kann;
b eine ganze Zahl von 0 bis 3 ist und von 0 bis 4 für die terminale Fructosyl-Einheit;
n eine ganze Zahl von 2 bis 499 ist, vorzugsweise von 2 bis 249, 2 bis 99, 2 bis 49, 9 bis 49, 14 bis 39, 19 bis 29 oder 19 bis 24,
wobei jede Einheit der Formel F(O-CO-NH-R²)_{b} gleich oder verschieden von jeder anderen Einheit der Formel F(O-CO-NH-R²)_{b} sein kann; und
der durchschnittliche Substitutionsgrad pro Saccharid-Einheit 0,02 bis 3,0 ist.

20. Verfahren oder Verwendung nach Anspruch 19, wobei jede Gruppe R¹ und R² aus Alkylgruppen mit 1 bis 25 Kohlenstoffatomen ausgewählt ist.

21. Verfahren oder Verwendung nach Anspruch 19, wobei jede Alkyl-Gruppe R¹ und R² eine lineare Alkylgruppe mit 1 bis 25 Kohlenstoffatomen oder eine verzweigte Alkylgruppe mit von 3 bis 25 Kohlenstoffatomen ist.

22. Verfahren oder Verwendung nach einem der Ansprüche 19 bis 21, wobei die Verbindung der Formel (I) ein polydisperses lineares oder geringfügig verzweigtes Inulin-N-Alkylurethan ist.

23. Verfahren oder Verwendung nach einem der Ansprüche 19 bis 22, wobei die Verbindung der Formel (I) ausgewählt ist aus der Gruppe bestehend aus Inulin-N-n-Octyl-Carbamaten, Inulin-N-n-dodecylcarbamaten und Inulin-N-n-Octadecylcarbamaten.

24. Zusammensetzung, umfassend ein Protein und ein Inulin-Derivat der Formel (I):
G(O-CO-NH-R¹)ₐ-[F(O-CO-NH-R²)_{b}]ₙ (I)
wobei:
G eine terminale Glucosyl-Einheit ist, bei der eine oder mehrere Hydroxyl-Gruppe(n) durch eine oder mehrere Gruppe(n) der Formel (O-CO-NH-R¹) substituiert sein kann/können;
R¹ ausgewählt ist aus der Gruppe, welche Alkylgruppen umfasst, und, wo mehr als eine (O-CO-NH-R¹) Gruppe an der Glucosyl-Einheit sind, wobei jede Gruppe R¹ gleich oder verschieden sein kann;
a eine ganze Zahl von 0 bis 4 ist;
F eine Fructosyl-Einheit ist, bei der eine oder mehrere Hydroxyl-Gruppe(n) durch eine oder mehrere Gruppe(n) der Formel (O-CO-NH-R²) substituiert sein kann/können;
R² ausgewählt ist aus der Gruppe, welche Alkylgruppen umfasst, und, wo mehr als eine (O-CO-NH-R²) Gruppe an der Fructosyl-Einheit sind, jede Gruppe R² gleich oder
verschieden sein kann;
b eine ganze Zahl von 0 bis 3 ist und von 0 bis 4 für die terminale Fructosyl-Einheit;
n eine ganze Zahl von 2 bis 499 ist, vorzugsweise von 2 bis 249, 2 bis 99, 2 bis 49, 9 bis 49, 14 bis 39, 19 bis 29 oder 19 bis 24,
wobei jede Einheit der Formel F(O-CO-NH-R²)_{b} gleich oder verschieden von jeder anderen Einheit der Formel F(O-CO-NH-R²)_{b} sein kann; und
der durchschnittliche Substitutionsgrad pro Saccharid-Einheit 0,02 bis 3,0 ist.

25. Zusammensetzung nach Anspruch 24, wobei die Verbindung der Formel (I) ein polydisperses lineares oder geringfügig verzweigtes Inulin N-Alkylurethan ist.

26. Zusammensetzung nach Anspruch 24 oder Anspruch 25, wobei die Verbindung der Formel (I) ausgewählt ist aus der Gruppe bestehend aus Inulin-N-n-Octyl-Carbamaten, Inulin-N-n-Dodecylcarbamaten und Inulin-N-n-Octadecylcarbamaten.

27. Zusammensetzung nach einem der Ansprüche 24 bis 26, wobei die molare Konzentration des Inulin-Derivats im Bereich des 0,1- bis 1000-fachen der molaren Konzentration des Proteins ist.

28. Zusammensetzung nach einem der Ansprüche 24 bis 27, die eine wässrige Lösung ist.

29. Zusammensetzung nach einem der Ansprüche 24 bis 28 in trockener Form.

30. Zusammensetzung nach einem der Ansprüche 24 bis 29, welche eine pharmazeutische Zusammensetzung ist.

## Revendications

1. Procédé de stabilisation d'une protéine, **caractérisé par** l'étape consistant à mettre en contact la protéine avec un dérivé de polymère de sucre, ledit dérivé de polymère de sucre n'étant pas une α-, β- ou γ-cyclodextrine, dans lequel le dérivé de polymère de sucre est un dérivé d'inuline dérivé par un ou plusieurs type(s) de groupe hydrocarbyle non polaire sélectionné parmi le groupe constitué d'un/de dérivé(s) d'alkyle linéaire et d'un/de dérivé(s) d'alkyle ramifié et dans lequel la protéine est stabilisée contre l'agrégation, le changement conformationnel et/ou la dégradation.

2. Procédé selon la revendication 1, **caractérisé en ce que** la protéine et le dérivé d'inuline sont mis en contact dans une solution aqueuse.

3. Procédé selon la revendication 2, comprenant en outre l'étape consistant à sécher la solution par dessiccation, déshydratation, évaporation ou lyophilisation.

4. Procédé selon l'une quelconque des revendications 1 à 3, dans lequel la protéine est stabilisée contre le changement conformationnel.

5. Procédé selon l'une quelconque des revendications 1 à 4, dans lequel la protéine est une protéine dénaturée.

6. Procédé selon l'une quelconque des revendications 1 à 4, dans lequel la protéine est une conformation native.

7. Procédé selon la revendication 6, dans lequel la protéine est stabilisée contre la dénaturation.

8. Procédé selon l'une quelconque des revendications 1 à 7, dans lequel la protéine est stabilisée contre l'agrégation.

9. Procédé selon l'une quelconque des revendications 1 à 8, dans lequel la protéine est protégée contre la dégradation.

10. Procédé selon l'une quelconque des revendications 1 à 9, dans lequel la concentration du dérivé d'inuline est dans la gamme de 0,1 à 1000 fois la concentration molaire de la protéine.

11. Procédé selon l'une quelconque des revendications 1 à 10, dans lequel le dérivé d'inuline est présent dans une concentration de 0,002 mg/ml à 100 mg/ml.

12. Procédé de traitement de protéine comprenant un procédé selon l'une quelconque des revendications 1 à 11.

13. Procédé selon la revendication 12, comprenant un procédé de repliement de protéine et/ou comprenant une ou plusieurs étape(s) de purification de protéine.

14. Procédé selon la revendication 13, dans lequel ladite/lesdites étape(s) de purification de protéine est/sont sélectionnée(s) parmi le groupe constitué de : dialyse, diafiltration, ultrafiltration et chromatographie.

15. Utilisation d'un dérivé d'inuline, qui est dérivé par un ou plusieurs type(s) de groupe hydrocarbyle non polaire sélectionné parmi le groupe constitué d'un/de dérivé(s) d'alkyle linéaire et d'un/de dérivé(s) d'alkyle ramifié, pour stabiliser une protéine contre le changement conformationnel et/ou la dégradation.

16. Utilisation selon la revendication 15, pour stabiliser une protéine dans une solution aqueuse.

17. Utilisation selon la revendication 15 ou la revendication 16, pour stabiliser une protéine dans une forme sèche.

18. Procédé ou utilisation selon l'une quelconque des revendications 1 à 17, dans lequel/laquelle le dérivé d'inuline possède un degré de polymérisation de 3 à 500, 3 à 250 ou 3 à 100, de préférence de 3 à 50, plus préférablement de 10 à 50, encore plus préférablement de 15 à 40, encore préférablement de 20 à 30.

19. Procédé ou utilisation selon l'une quelconque des revendications 1 à 18, dans lequel/laquelle le dérivé d'inuline est un composé selon la formule (I) :
G (O-CO-NH-R¹)ₐ-[F(O-CO-NH-R²)_{b}]ₙ (I)
dans laquelle :
G est une unité de glucosyle terminale dans laquelle un ou plusieurs groupes hydroxyle de celle-ci peuvent être substitués par un groupe ou des groupes de formule (O-CO-NH-R¹) ;
R¹ est sélectionné parmi le groupe comprenant des groupes alkyle et, où il y a plus d'un groupe (O-CO-NH-R¹) sur l'unité de glucosyle, chaque groupe R¹ peut être identique ou différent ;
a est un entier de 0 à 4 ;
F est une unité de fructosyle dans laquelle un ou plusieurs groupes hydroxyle de celle-ci peuvent être substitués par un groupe ou des groupes de formule (O-CO-NH-R²) ;
R² est sélectionné parmi le groupe comprenant des groupes alkyle et, où il y a plus d'un groupe (O-CO-NH-R²) sur l'unité de fructosyle, chaque groupe R² peut être identique ou différent ;
b est un entier de 0 à 3 et de 0 à 4 pour l'unité de fructosyle terminale ;
n est un entier de 2 à 499, de préférence de 2 à 249, 2 à 99, 2 à 49, 9 à 49, 14 à 39, 19 à 29, ou 19 à 24,
chaque unité de formule F(O-CO-NH-R²)_{b} peut être identique ou différente de toute autre unité de formule F(O-CO-NH-R²)_{b} ; et
le degré moyen de substitution par unité de saccharide est de 0,02 à 3,0.

20. Procédé ou utilisation selon la revendication 19, dans lequel/laquelle chaque groupe R¹ et R² est sélectionné parmi des groupes alkyle possédant d'1 à 25 atomes de carbone.

21. Procédé ou utilisation selon la revendication 19, dans lequel chaque groupe alkyle R¹ et R² est un groupe alkyle linéaire possédant d'1 à 25 atomes de carbone ou un groupe alkyle ramifié possédant de 3 à 25 atomes de carbone.

22. Procédé ou utilisation selon l'une quelconque des revendications 19 à 21, dans lequel/laquelle le composé de formule (I) est un N-alkyluréthane d'inuline linéaire ou légèrement ramifié polydispersé.

23. Procédé ou utilisation selon l'une quelconque des revendications 19 à 22, dans lequel/laquelle le composé de formule (I) est sélectionné parmi le groupe constitué de N-n-octyl-carbamates d'inuline, de N-n-dodécylcarbamates d'inuline et de N-n-octadécylcarbamates d'inuline.

24. Composition comprenant une protéine et un dérivé d'inuline de formule (I) :
G (O-CO-NH-R¹)ₐ-[F(O-CO-NH-R²)_{b}]ₙ (I)
dans laquelle :
G est une unité de glucosyle terminale dans laquelle un ou
plusieurs groupes hydroxyle de celle-ci peuvent être substitués par un groupe ou des groupes de formule (O-CO-NH-R¹) ;
R¹ est sélectionné parmi le groupe comprenant des groupes alkyle et, où il y a plus d'un groupe (O-CO-NH-R¹) sur l'unité de glucosyle, chaque groupe R¹ peut être identique ou différent ;
a est un entier de 0 à 4 ;
F est une unité de fructosyle dans laquelle un ou plusieurs groupes hydroxyle de celle-ci peuvent être substitués par un groupe ou des groupes de formule (O-CO-NH-R²) ;
R² est sélectionné parmi le groupe comprenant des groupes alkyle et, où il y a plus d'un groupe (O-CO-NH-R²) sur l'unité de fructosyle, chaque groupe R² peut être identique ou différent ;
b est un entier de 0 à 3 et de 0 à 4 pour l'unité de fructosyle terminale ;
n est un entier de 2 à 499, de préférence de 2 à 249, 2 à 99, 2 à 49, 9 à 49, 14 à 39, 19 à 29, ou 19 à 24,
chaque unité de formule F(O-CO-NH-R²)_{b} peut être identique ou différente de toute autre unité de formule F(O-CO-NH-R²)_{b} ; et
le degré moyen de substitution par unité de saccharide est de 0,02 à 3,0.

25. Composition selon la revendication 24, dans laquelle le composé de formule (I) est un N-alkyluréthane d'inuline linéaire ou légèrement ramifié polydispersé.

26. Composition selon la revendication 24 ou la revendication 25, dans laquelle le composé de formule (I) est sélectionné parmi le groupe constitué de N-n-octyl-carbamates d'inuline, de N-n-dodécylcarbamates d'inuline et de N-n-octadécylcarbamates d'inuline.

27. Composition selon l'une quelconque des revendications 24 à 26, dans laquelle la concentration molaire du dérivé d'inuline est dans la gamme de 0,1 à 1000 fois la concentration molaire de la protéine.

28. Composition selon l'une quelconque des revendications 24 à 27, qui est une solution aqueuse.

29. Composition selon l'une quelconque des revendications 24 à 28 sous forme sèche.

30. Composition selon l'une quelconque des revendications 24 à 29, qui est une composition pharmaceutique.
